# EUROPEAN PATENT SPECIFICATION

(11) **EP 3 355 848 B1**
(45) Date of publication and mention of the grant of the patent: **28.10.2020**
(21) Application number: 16766054.7
(22) Date of filing: 08.09.2016
(51) Int. Cl.: A61K 8/24, A61K 8/34, A61K 8/365, A61K 8/42, A61K 8/44, A61K 8/46, A61Q 17/00

(54) **ALCOHOL-BASED SKIN SANITIZER HAVING MICROBICIDAL PROPERTIES**
HAUTREINIGER AUF ALKOHOLBASIS MIT MIKROBIZIDEN EIGENSCHAFTEN
DESINFECTANT ALCOOLIQUE POUR LA PEAU AVEC DES PROPRIETES MICROBICIDES

(30) Priority: 30.09.2015 US 201562234933 P
(43) Date of publication of application: 08.08.2018
(73) Proprietor: Deb IP Limited, Denby, Derbyshire DE5 8JZ (GB)
(72) Inventor: GRASCHA, Pierre, 51350 Cormontreuil (FR); ORMANDY, Kevin Anthony, Sheffiled Yorkshire S11 9NT (GB); LATASTE, Sylvain, 92160 Antony (FR)
(74) Representative: Elsy, David
(86) International application number: PCT/GB2016/052782
(87) International publication number: WO 2017/055797

(56) References cited:
- US-A1- 2007 275 929
- US-A1- 2007 281 999
- US-A1- 2009 018 213
- US-A1- 2014 275 255

## Description

### FIELD

This disclosure relates to fast-acting disinfecting alcohol-based formulations for use on skin.

### BACKGROUND

Hand hygiene is a key fact in the fight against infections associated with care and transmission of pathogenic agent. However, in hospital, compliance of hand cleaning with water and soap is not as important as it should be because there are a lot of constraints (requires hand washing, rinsing and drying followed by hand sanitization); it is the reason why the industry has began to develop some hydro-alcoholic formulations.

Marketed in the 90s, these products were first not appreciated by hygienists who were just promoting compliance of hand washing. Since, quite a few studies have demonstrated that the use of gentle soap, followed by alcohol-based hand sanitizer, reduces the occurrence of occupational hand dermatitis. Today, alcohol-based hand sanitizers are the preferred method for hand hygiene and pre-operatory hand sanitization, in complement of soap.

Alcohol-based hand sanitizers are generally composed of 20-30% of water (which is absolutely necessary for the denaturation of the bacterial proteins), 60-80% of alcohols (ethanol and/or isopropanol and/or n-propanol) and they contain sometimes some extra biocidal agents (i.e. chlorhexidine, quaternary ammoniums, phenolics...) in order to strengthen their initial antimicrobial spectrum of efficacy. These extra biocides may have skin keratolytic and/or irritating properties (some cases of allergy are also described in the specialized literature) when long-term used; therefore, they are not suitable in leave-on products from a purely toxicological point of view.

Without biocidal additives, alcohols have broad-spectrum activity against vegetative gram positive and gram negative bacteria, mycobacteria, yeasts, and enveloped viruses. They are slow-active on fungi (required contact ≥ 5 minutes) and they also may be considered as inactive on non-enveloped viruses (also called hydrophilic or naked viruses) within acceptable contact time for the purpose of fast skin hygiene disinfection (1 minute or less). In order to achieve efficacy on naked viruses within one minute or less, some currently marketed hand sanitizers involve 80 to 90% w/w of ethanol and/or n-propanol and/or isopropanol as well as some kill-boosters such as fatty alcohols, surfactants, or glycols. However, increasing the concentration of alcohol to more than 80% may lead to a loss of bactericidal efficacy; 20 to 30% of water being necessary to make the alcohol penetrate into the bacterial cells and coagulate their cytoplasmic proteins. Also, high concentration of alcohol is not suitable in terms of skin tolerance as it increases the trans-epidermal-water-loss and the related skin dehydration. Extra biocidal additives such as quaternary ammoniums, chlorhexidine, triclosan, p-chloro-m-xylenol, iodine, etc, are not suitable either because of the risk of skin irritation and/or sensitization; these additives remaining on the skin all day long and every day for staff working in the medical and the food sectors.

### SUMMARY

An object of the present disclosure is to provide an alcohol-based composition or formulation, preferably containing from about 60 to about 70% w/w alcohol for use as a hand sanitizer, and having efficacy on most families of pathogenic micro-organisms, including non-enveloped viruses.

Typically, the formulation is free of any co-biocide in addition to the alcohol(s). This reduces the risk of skin irritation or allergy.

In order to improve the *in vitro* efficacy of the invention on bacteria, yeast and non-enveloped viruses as well as the *in vivo* efficacy on bacteria, formulation strategy was based on increasing the number of cell-components the ingredients of the composition can target without involving biocidal co-actives. The ultimate composition obtained combines alcohol(s) with ingredients in a synergistic formulation having satisfactory skin compatibility, no or little intrinsic antimicrobial properties and the potential to increase the spectrum of activity as well as the germ kill rate of the formulation due to their chemical action on the microbial cell components.

The present disclosure provides low or high alcohol content compositions, with high level of antimicrobial efficacy, which contain a surfactant/wetting agent as well as a disinfectant/solvent/carrier and that causes very little drying to the skin or the hands of the user and is able to be dispensed as a liquid, as a gel, as a foam or dispensed from a pressurized system.

Accordingly, an aspect of the present disclosure provides a liquid composition for personal hygiene. The composition can be a foaming formulation. The composition can be a thickened formulation.

An aspect of the present disclosure is a skin disinfectant composition or formulation, comprising:
(a) an amount of 60-70% w/w of a C2-C5 aliphatic alcohol, wherein the alcohol is ethanol;
(b) one or more chelating agent(s) present in an amount of from about 0.1 to about 5 % w/w of the composition;
(c) one or more hydrotrope(s), present in an amount of from about 0.1 to 5.0 % w/w of the composition;
(d) one or more inorganic acid(s) and/or one or more organic acid(s) present in an amount of from about 0.01 to about 5 % w/w of the composition, selected to adjust the pH of the composition in a range from about pH 2 to about pH 6;
(e) one or more C10 to C16 alkylbenzene sulfonate(s) present in an amount of from about 0.1 to 1.0 % w/w of the composition;
(f) urea, ammonium sulphate or a combination therof present in an amount of from about 0.01 to about 10 % w/w of the composition;
(g) one or more sesquiterpenoid(s) present in an amount of from about 0.01 to about 1 % w/w of the composition;
(h) one or more of skin moisturizing, conditioning and emollient agent(s) present in an amount of from about 0.01 to about 10 % w/w of the composition; and
(i) water is present in an amount to balance the total composition to 100 % w/w.

An aspect of the disclosure is a composition formulated as a foaming composition. Such foaming formulations include a surfactant present in an amount of about 1 to 5 % w/w of the composition.

Another aspect of the present disclosure is one in which the composition is thickened by a rheology modifier present in amount of from about 0.01 to about 10 % w/w of the composition.

### BRIEF DESCRIPTION OF THE DRAWINGS

Embodiments will hereinafter be described with reference to the accompanying drawings, which have not necessarily been drawn to scale.
**Figure** 1 shows graphically results bacterial obtained using *in vitro* tests, with a contact time of 15 seconds and a concentration in product of 80% vol/vol. Figure (a) shows the results of bactericidal tests: ASTM E2315-03 time-kill test on *Escherichia coli* ATCC 11229. Figure (b) shows the test results obtained with *Staphylococcus aureus* ATCC 6538;
**Figure 2** shows graphically results obtained using i*n vitro* yeasticidal tests: EN13624 norm on *Candida albicans* ATCC 10231, with a contact time of 15 seconds and a concentration in product of 80% vol/vol;
**Figure 3** shows graphically results obtained using *in vitro* virucidal tests: EN14476 norm on Poliovirus, with a contact time of 30 seconds and a concentration in product of 80% vol/vol; and
**Figure 4** shows graphically *in vivo* bactericidal tests: EN1500 norm on *Escherichia coli* K12, involving 5 subjects, a contact time of 30 seconds and a volume of product of 3ml.

### DETAILED DESCRIPTION

In an aspect, the invention provides a fast acting hydro-alcoholic skin disinfecting (bactericidal, fungicidal and virucidal) solution that includes an amount of 60-70% w/w of a C2 to C5 aliphatic alcohol, wherein the alcohol is ethanol, and preferably present at a concentration of 70 % w/w of the composition.

The composition includes at least one chelating agent present in a concentration of from 0.1 to 5 % w/w. The chelating agent component can be one or more chelating agents chosen from glutamic acid, phosphonic acids having 1 to 5 phosphonic acid groups, e.g. 1-hydroxyethylidene-1,1,-diphosphonic acid, amino tri(methylene phosphonic acid), diethylenetriaminepenta(methylene phosphonic acid), 2-hydroxy ethylimino bis(methylene phosphonic acid), and ethylene diamine tetra(methylene phosphonic acid), sodium tripolyphosphate, ethylenediaminetetraacetic acid (EDTA), diethylenetriaminepentaacetic acid (DTPA), N-(hydroxyethyl)-ethylenediami-netriacetic acid (HEDTA), 2-hydroxyethyliminodiacetic acid (HEIDA), iminodisuccinic acid, polyaspartic acid, tetrasodium glutamate diacetate, and so can be any combination of any of the foregoing. A preferred chelating agent is tetrasodium glutamate diacetate, and preferably the chelating agent(s) component of the composition is present in an amount of 0.4 % w/w.

The composition includes a component that is at least one hydrotrope present in an amount of from 0.1 to 5.0 % w/w of the composition or formulation. The one or more hydrotrope can be chosen from xylene sulfonic acid, sodium xylene sulfonate, ammonium xylene sulfonate, calcium xylene sulfonate, potassium xylene sulfonate, toluene sulfonic acid, sodium toluene sulfonate, ammonium toluene sulfonate, cumene sulfonic acid, sodium cumene sulfonate, ammonium cumene sulfonate, phosphate polyether ester, alkyldiphenyloxide disulfonate, and any combination of any of the foregoing. A preferred hydrotrope is cumene sulfonic acid or its salt. A preferred amount of the hydrotrope(s) componenent is 0.5 % w/w.

The composition includes at least one acid. The acid(s) can be inorganic and/or organic acids. The acid(s) component is present in an amount ranging from about 0.01 to about 5 wt/wt % of the composition and the one or more acid(s) is selected to adjust the pH of the formulation in a range from pH 2 to pH 6. Acid(s) can be selected from alpha-hydroxy-acids (e.g. citric acid, lactic acid, tartric acid, malic acid, glycolic acid), maleic acid, propanoic, butanoic, pentanoic, hexanoic, heptanoic, octanioc mono-, di- and tri-acids, beta-hydroxy-acids, phosphoric acid, and any combination thereof. A preferred acid is phosphoric acid, and preferably the formulation has a pH of 4.5.

The composition includes one or more alkylbenzene sulfonate chosen from those having 10 to 16 carbons present in an amount of from 0.1 to 1.0 % w/w of the composition. The one or more alkylbenzene sulfonate(s) can be chosen from sodium decylbenzene sulfonate, sodium dodecylbenzene sulfonate, sodium tridecylbenzene sulfonate, and so can be a combination of any of these. A preferred alkylbenzene sulfonate is alkylbenzene sulfonate and a preferred concentration of the alkylbenzene sulfonate(s) is 0.1 % w/w.

The composition includes one or more chaotrope(s) present in a concentration of from 0.01 to 10 % w/w of the composition. The chaotrope is the denaturant urea or ammonium sulphate and a preferred concentration of the chaotrope(s) component is between 0.1 and 5 % w/w, more preferably 1 % w/w of the composition.

A composition of the present disclosure also includes one or more sesquiterpenoid(s), this component being present as 0.01 to 1 % w/w of the composition. In an embodiment, the at least one sesquiterpenoid is chosen from nerolidol, farnesol, bisabolol, and apritone, and can be a combination of any of these. A preferred concentration of the sesquiterpenoid(s) component is 0.1 % w/w of the composition.

The composition also includes at least one skin moisturizing and/or conditioning and/or emollient agent in a concentration of from 0.01 to 10 % w/w of the formulation. Such ingredients can be chosen from polyols and glycols such as glycerol, polyglycerol, sorbitol, mannitol, erythritol, xylitol, arabitol, ribitol, dulcitol, lactitol, maltitol, propylene glycol, polyethylene glycol, hexylene glycol, butylene glycol, allantoin, phospholipids such as soya lecithin, ceramides, essential fatty acids such as linolenic acid, gamma-linolenic acid, linoleic acid, and gamma-linoleic acid, vitamins such as d-panthenol and tocopherols, cationics such as quaternised gums quaternised guar gum, dihydroxypropyl PEG-5 linoleammonium chloride, behentrimonium chloride, and polyquaterniums, sugar derivatives such as glucose-ethers such as methyl gluceth-20, glycerol esters such as glyceryl oleate, glyceryl palmate, glyceryl cocoate, and glyceryl undecylenate, and vegetable extracts such as *Matricaria chamomilla, Aloe vera,* and *Glycyrrhiza glabra* L. A prefered moisturizer is d-panthenol, and a preferred concentration is in the range of from 0.01 to 1 % w/w of the composition. A preferred concentration of the one or more at skin moisturizing and/or conditioning and/or emollient agent is 0.5 % w/w of the composition.

As mentioned previously, water is present in an amount to balance the total composition to 100% w/w.

A composition of the invention can also be prepared as foam that contains at least one surfactant. For such foaming versions, the one or more surfactant(s) can be chosen from ethoxylated dimethicone having a "comb structure" i.e., having a functional group located at an internal position of the molecule, or a "terminal structure" in which the functional group is located at the ends of the molecule. Examples are 3-(3-Hydroxypropyl)-heptamethyltrisiloxane, polyether-modified polysiloxane or a polysiloxane betaine, alkenyl esters or diesters of sulfosuccinic acid in which the alkyl or alkenyl groups independently contain from six to eighteen carbon atoms, and an alkali metal, triethanolamine, sodium, ammonium, calcium or magnesium, C8 to C18 alkyl ether sulfates, or C8-C18 alkyl amine oxide, or alkylpolyglusoside, or water-soluble resin such as non-ionic polymers of ethylene oxide with molecular weight ranging from 100,000 to 4000,000 Daltons. A preferred surfactant is Bis-PEG-12 dimethicone at 1 to 5 % w/w of the composition, and a preferred concentration is 1.00 % w/w. A preferred water-soluble resin is polyethylene oxide with a molecular weight of 1000,000 at a preferred concentration of 0.05 % w/w of the composition.

A composition of the invention can be thickened, containing at least one rheology modifier at a concentration of from 0.01 to 10 % w/w of the composition. The one or more rheology modifier can be chosen from acrylates/C10-30 alkyl acrylate crosspolymer, carbomer, xanthan gum, guar gum, cellulose ether, quaternised guar gum, alginate and bentonite, and combination thereof. A preferred range is from about 0.1 to 10 % w/w of the composition. A preferred thickener is acrylates/C10-30 alkyl acrylate crosspolymer or carbomer at 0.1 and 1% w/w of the gel formulation.

Preferred additives are described in greater detail below.

### Cumene sulfonic acid

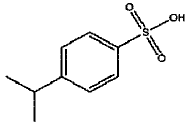

Cumene sulfonic acid, also known as benzenesulfonic acid, (1-methylethyl) cumene sulfonic acid (CAS: 16066-35-6), is a hydrotrope; as such it should help to solubilise hydrophobic membranes and envelopes of micro-organisms. Therefore, in the present invention, cumene sulfonic acid is believed to improve the bactericidal and yeasticidal properties of the involved alcohol(s).

### Tetrasodium glutamate diacetate

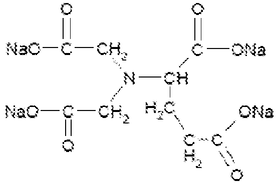

Tetrasodium glutamate diacetate, also known as tetrasodium N,N-bis(carboxylatomethyl)-L-glutamate (CAS: 51981-21-6), is a biodegradable and safe as a cosmetic chelating agent. It is a substitute for EDTA.

Bacteria require metal ions to satisfy the specific requirements of metal-enzyme and cell-wall structural components. Chelators are able to increase the permeability of the bacterial cell wall by sequestering the necessary metals (Fe²⁺ in particular). They also can capture the metal ions (Mg²⁺ in particular) acting as cofactors for the DNA synthesis and involved in the LipoPolySaccharide's cohesion.

In the present invention, tetrasodium glutamate diacetate is believed to improve the killing rate on gram negative bacteria mainly.

### Nerolidol

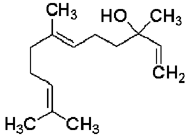

A natural sesquiterpene, also called peruviol or 3,7,11-trimethyl-1,6,10-dodecatrien-3-ol (CAS: 7212-44-4), nerolidol is found in the essential oils of many types of plants and flowers, and with potential bactericidal and fungicidal properties.

The antimicrobial activity of nerolidol has been documented in various studies. A study (1) consisting of evaluating the antibacterial effects of three terpene-alcohols (farnesol, nerolidol and plaunotol) on *Staphylococcus aureus,* focusing on the leakage of K⁺ ions and toxicity over time, suggested that the terpene alcohols might act on cell membranes. The antibacterial activity reflected the initial rate of leakage of K⁺ ions, suggesting that damage to cell membranes might be one of the major modes of action of these terpene alcohols. The results also demonstrated that the initial rate of leakage and the amount of leaked K⁺ ions are useful as indices of the antibacterial activities of hydrophobic compounds.

In another study (2), sesquiterpenoids nerolidol, farnesol, bisabolol, and apritone were investigated for their abilities to enhance bacterial permeability and susceptibility to exogenous antimicrobial compounds. Initially, it was observed by flow cytometry that these sesquiterpenoids promoted the intracellular accumulation of the membrane-impermeant nucleic acid stain ethidium bromide by live cells of *Lactobacillus fermentum,* suggesting that enhanced permeability resulted from disruption of the cytoplasmic membrane. In disk diffusion assays, treatment with low concentrations (0.5 to 2 mM) of nerolidol, bisabolol, or apritone enhanced the susceptibility of *Staphylococcus aureus* to ciprofloxacin, clindamycin, erythromycin, gentamicin, tetracycline, and vancomycin. Nerolidol and farnesol also sensitized Escherichia coli to polymyxin B.

A more recent study (3) allowed elucidating the antifungal activities of eugenol and nerolidol isolated from Japanese cypress oil in a guinea pig model infected by *Microsporum gypseum.* A minimal inhibitory concentration (MIC), skin lesion scoring, hair culture and histopathologic examination of skin tissues were performed to evaluate the antifungal effect of these oils. The MICs of eugenol, nerolidol and econazole (positive control) were 0.01-0.03% and 0.5-2% and 4-16 µg/ml, respectively. Based on these MICs, eugenol and nerolidol were adjusted to 10% concentration with a base of Vaseline® petroleum jelly and were applied topically to the skin lesion infected with *M. gypseum* daily for 3 weeks. Both eugenol and nerolidol were clinically effective at improving the lesion during the first week of application, as determined by skin lesion scoring. Nerolidol improved the skin lesions infected by *M. gypseum,* but eugenol did not, as determined in the hair culture test. Histopathologic examination revealed that the eugenol- and nerolidol-treated groups had a lower degree of hyperkeratosis and inflammatory cell infiltration than the positive control. Taken together, these results suggest that eugenol and nerolidol could apply supplementary antifungal agents.

In the present disclosure, nerolidol is believed to improve the killing rate on gram negative bacteria.

### Urea

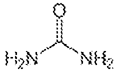

Urea, also known as carbamide, carbonyl diamide, carbonyldiamine, diaminomethanal, or diaminomethanone (CAS: 57-13-6), is a powerful chaotrope used for denaturing proteins; it has the potential to disrupt the non-covalent bonds of secondary and tertiary structures of cellular proteins and enzymes of bacteria, fungi and viruses; denatured proteins will unfold into long polypeptide chains without involving the hydrolysis or destruction of the peptide bonds.

In the present disclosure, urea is believed to improve the killing rate on gram negative bacteria, yeasts, and non-enveloped viruses.

### Dodecylbenzene sulfonic acid

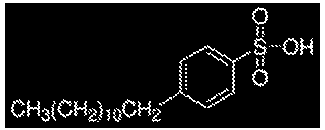

Dodecylbenzene sulfonic acid is also known as Alky-aryl-sulfonic acid; Alky-benzene-sulfonic acid; 4-dodecyl-benzene sulfonic acid; p-dodecyl-benzene sulfonic acid (CAS: 121-65-3). The choice of such an anionic compound is based on theoretical approach: most aminoacids composing the proteins of viral capsids have their isoelectric points at around 5-6, therefore, at acidic pH these amino acids become cationic. At pH < 5 and in the presence of anionic compounds the aminoacids should precipitate because they have reached the point of minimum water solubility.

Anionic surfactants should also disrupt the intramolecular ionic bonds of proteins and solubilise bacterial, fungal and viral lipid membranes or envelop.

In the present disclosure, dodecyl benzene sulfonic acid is believed to improve the killing rate on gram negative bacteria, yeasts, and enveloped as well as non-enveloped viruses.

Except where explicitly indicated, all numbers in this description indicating amounts or ratios of materials, conditions of reaction; physical properties of materials and/or use; dimensions and dimension ratios, are to be understood as modified by the word "about".

The term "comprising" is meant not to be limiting to any subsequently stated elements but rather to encompass non-specified elements of major or minor functional importance. In other words the listed steps, elements or options need not be exhaustive. Whenever the words "including" or "having" are used, these terms are meant to be equivalent to "comprising" as defined above. It should be noted that in specifying any range of concentration or amount, any particular upper concentration or amount can be associated with any particular lower concentration or amount. Reported ranges are inclusive of their endpoints.

All parts, percentages, ratios, and proportions referred to in this specification, including the claims, are by weight unless otherwise indicated.

The following Examples will more fully illustrate the embodiments of this disclosure. The examples are not intended to limit the scope of the invention in any manner.

### EXAMPLES

| **LIQUID FORMULATION** | | **Referenc e alcohol** | **Reference hand santizer** | **Liquid Test formulatio n** | **Foaming Test formulatio n** | **Thickened Test formulatio n** |
|---|---|---|---|---|---|---|
| **Ingredients** | **CAS** | | | | | |
| **Purified water** | 7732-18-5 | Up to 100% | Up to 100% | Up to 100% | Up to 100% | Up to 100% |
| **D-Panthenol** | 81-13-0 | | 0.50 | 0.50 | 0.50 | 0.50 |
| **Dihydroxypropyl PEG-5** | 168677-75-6 | | 0.05 | 0.05 | 0.05 | 0.05 |
| **Linoleamonium Chloride** | | | | | | |
| **Bis-PEG-12 Dimethicone** | 102783-01-7 | | | | 1.00 | |
| **Polyethylene oxide** | 25322-68-3 | | | | 0.05 | |
| **Carbomer** | 9003-01-4 | | | | | 0.40 |
| **Aminomethyl propanol** | 124-68-5 | | | | | 0.40 |
| | | | | | | |
| **Ethanol** | 64-17-5 | 70.00 | 65.00 | 70.00 | 70.00 | 70.00 |
| **N-Propanol** | 71-23-8 | | 10.00 | | | |
| **Urea** | 57-13-6 | | | 1.00 | 1.00 | 1.00 |
| **Nerolidol** | 7212-44-4 | | | 0.10 | 0.10 | 0.10 |
| **Tetrasodium Glutamate Diacetate** | 51981-21-6 | | | 0.40 | 0.40 | 0.40 |
| **Sodium dodecylbenzene sulfonate** | 68081-81-2 | | | 0.10 | 0.10 | 0.10 |
| **Cumene sulfonic acid** | 28631-63-2 | | | 0.50 | 0.50 | 0.50 |
| **Phosphoric acid (to adjust final pH at 4-5)** | 7664-38-2 | 0.10 to 0.50 | | | | |

### Assessment of Microbiocidal Efficacy

All above formulations were subject to preliminary *in vitro* and *in vivo* testing in order to pre-evaluate their level of microbiocidal efficacy on gram positive and gram negative bacteria, as well as yeast and non-enveloped virus.

All formulations were tested against one reference 70% w/w ethanol aqueous solution and one market alcohol-based hand sanitizer containing ethanol 65% w/w and n-propanol 10% w/w.

### Test Results

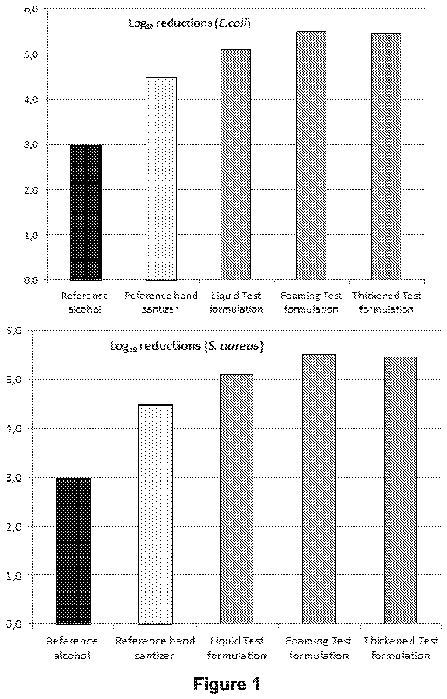

*In vitro* bactericidal tests: ASTM E2315-03 time-kill test on *Escherichia coli* ATCC 11229 and *Staphylococcus aureus* ATCC 6538, with a contact time of 15 seconds and a concentration in product of 80% vol/vol. See Figure 1.

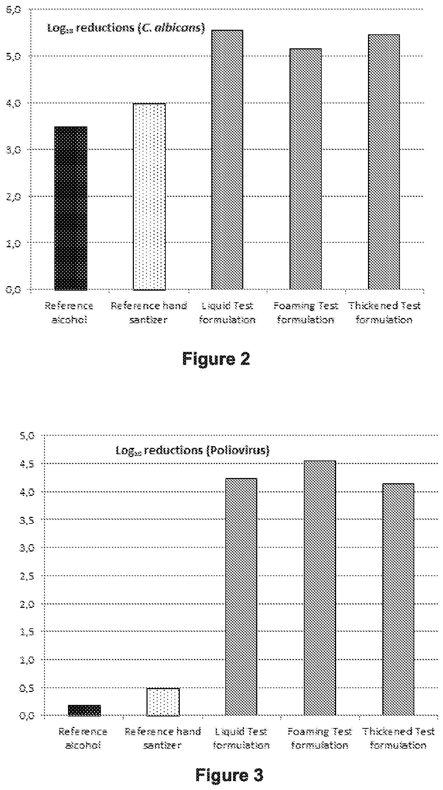

*In vitro* veasticidal tests: EN13624 norm on *Candida albicans* ATCC 10231, with a contact time of 15 seconds and a concentration in product of 80% vol/vol. See Figure 2.

*In vitro* virucidal tests: EN14476 norm on Poliovirus, with a contact time of 30 seconds and a concentration in product of 80% vol/vol. See Figure 3.

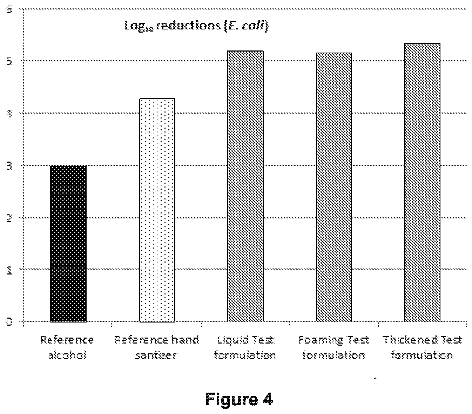

For *in vivo* bactericidal tests: EN1500 norm on *Escherichia coli* K12, involving 5 subjects, a contact time of 30 seconds and a volume of product of 3ml. See Figure 4.

For all of the above tests, the test-formulations were more effective on bacteria (*Escherichia coli and Staphylococcus aureus*)*,* the yeast (*Candida albicans*) and the non-enveloped virus (Poliovirus) than the reference 70° ethanol solution and the reference market hand sanitiser. This supports the proposition that the additives have increased the microbiocidal properties of the alcohol (ethanol at 70% w/w).

### Preliminary assessment of skin irritation potential

Prior to performing *in vivo* or *in vitro* skin irritation tests, skin irritation scores were calculated in accordance with the Directive 1999/45/EC ('Classification, Packaging and Labelling of Dangerous Preparations') of the European Parliament and of the Council of 31 May 1999.

According to the directive, none of the above formulations is classified as « irritating to skin » (the limit being '1') and because the calculated scores are very low (all < 0.1), they may be considered as very mild to the skin.

An *in vivo* study of acute skin compatibility has also been conducted on 11 subjects and using the 48-hours a semi-occlusive patch-test method. The most concentrated formulation (#8) was tested pure and was classified as "non-irrating to the skin" as the obtained mean irritation index (M.I.I.) was zero.

### References

(1) Yoshihiro Inouea, Akiko Shiraishia, Toshiko Hadaa, Kazuma Hirosea, Hajime Hamashimaa, Jingoro Shimada, "The antibacterial effects of terpene alcohols on Staphylococcus aureus and their mode of action", FEMS microbiology letters (FEMS microbiol. lett.) 2004, 237(2), pp. 325-331.
(2) Byron F. Brehm-Stecher and Eric A. Johnson, "Sensitization of S. aureus and E. coli to Antibiotics by the Sesquiterpenoids Nerolidol, Farnesol, Bisabolol, and Apritone", Antimicrob Agents Chemother. 2003 October; 47(10), pp 3357-3360.
(3) Sook-Jin Lee, Je-Ik Han, Geun-Shik Lee, Mi-Jin Park, In-Gyu Choi, Ki-Jeong Na' Eui-Bae Jeung, "Antifungal Effect of Eugenol and Nerolidol against Microsporum gypseum in a Guinea Pig Model', Biological & Pharmaceutical Bulletin, Vol. 30 (2007), No. 1, p 184.

## Claims

1. A skin disinfectant composition, comprising:
(a) an amount of 60-70% w/w of a C2-C5 aliphatic alcohol, wherein the alcohol is ethanol
(b) one or more chelating agent(s) present in an amount of from 0.1 to 5 % w/w of the composition;
(c) one or more hydrotrope(s), present in an amount of from 0.1 to 5.0 % w/w of the composition;
(d) one or more inorganic acid(s) and/or one or more organic acid(s) present in an amount of from 0.01 to 5 % w/w of the composition, selected to adjust the pH of the composition in a range from about pH 2 to about pH 6;
(e) one or more C10 to C16 alkylbenzene sulfonate(s) present in an amount of from 0.1 to 1 .0 % w/w of the composition;
(f) urea, ammonium sulphate or a combination thereof present in an amount of from 0.01 to 10 % w/w of the composition;
(g) one or more sesquiterpenoid(s) present in an amount of from 0.01 to 1 % w/w of the composition;
(h) one or more of skin moisturizing, conditioning and emollient agent(s) present in an amount of from 0.01 to 10 % w/w of the composition; and
(i) water is present in an amount to balance the total composition to 100 % w/w.

2. The composition of claim 1, wherein said one or more chelating agent(s) is glutamic acid, one or more phosphonic acid having 1 to 5 phosphonic acid groups, or a mixture of any of the foregoing; preferably wherein said one or more chelating agent(s) is 1-hydroxyethylidene-1,1,-diphosphonic acid, amino tri(methylene phosphonic acid), diethylenetriaminepenta(methylene phosphonic acid), 2-hydroxy ethylimino bis(methylene phosphonic acid), ethylene diamine tetra(methylene phosphonic acid), sodium tripolyphosphate, ethylenediaminetetraacetic acid (EDTA), diethylenetriaminepentaacetic acid (DTPA), N-(hydroxyethyl)-ethylenediami- netriacetic acid (HEDTA), 2-hydroxyethyliminodiacetic acid (HEIDA), iminodisuccinic acid, polyaspartic acid, tetrasodium glutamate diacetate, or a mixture of any of the foregoing.

3. The composition of claim 2, wherein said one or more chelating agent(s) is tetrasodium glutamate diacetate.

4. The composition of any one of claims 1 to 3, wherein said one or more hydrotrope is xylene sulfonic acid, sodium xylene sulfonate, ammonium xylene sulfonate, calcium xylene sulfonate, potassium xylene sulfonate, toluene sulfonic acid, sodium toluene sulfonate, ammonium toluene sulfonate, cumene sulfonic acid, sodium cumene sulfonate, ammonium cumene sulfonate, phosphate polyether ester, alkyldiphenyloxide disulfonate, a salt of any of the foregoing or a mixture of any of the foregoing;
preferably wherein said one or more hydrotrope is cumene sulfonic acid or its salt.

5. The composition of any one of claims 1 to 4, wherein said one or more inorganic acid(s) and/or one or more organic acid(s) is one or more alpha- hydroxy-acid, maleic acid, propanoic acid, butanoic acid, pentanoic acid, hexanoic acid, heptanoic acid, a mono-octanoic acid, a di-octanoic acid, a tri-octanoic acid, one or more beta-hydroxy-acid(s), phosphoric acid or a combination of any of the foregoing;
preferably wherein said one or more alpha-hydroxy- acid is citric acid, lactic acid, tartric acid, malic acid, glycolic acid or a combination of any of the foregoing.

6. The composition of claims 1 to 5, wherein said one or C10 to C16 alkylbenzene sulfonate is sodium decylbenzene sulfonate, sodium dodecylbenzene sulfonate, sodium tridecylbenzene sulfonate or a combination of any of the foregoing.

7. The composition of any one of claims 1 to 6, wherein said one or more sesquiterpenoid(s) is nerolidol, farnesol, bisabolol, apritone or a combination of any of the foregoing.

8. The composition of claim 7, wherein said one or more sesquiterpenoid(s) is nerolidol.

9. The composition of any one of claims 1 to 8, wherein said one or more skin moisturizing and/or conditioning and/or emollient agent(s) is one or more polyol(s) and glycol(s), one or more phospholipid(s), one or more essential fatty acid(s), one or more vitamin(s), one or more cationic compound(s), one or more sugar derivative(s), one or more vegetable extract(s) or a combination of any of the foregoing.

10. The composition of claim 9, wherein said one or more phospholipid(s) is soya lecithin, a ceramide or a combination thereof;
and/or wherein said one or more essential fatty acid(s) is linolenic acid, gamma-linolenic acid, linoleic acid, gamma-linoleic acid or a combination of any of the foregoing;
and/or wherein said one or more vitamin(s), is d-panthenol, a tocopherol or a combination thereof;
and/or wherein said one or more cationic compound(s) is a quaternised gum, a quaternised guar gum, dihydroxypropyl PEG-5 linoleammonium chloride, behentrimonium chloride, a polyquaternium or a combination of any of the foregoing;

11. The composition of any one of claims 9 to 10, wherein said one or more sugar derivative(s) is one or more glucose-ether(s), one or more glycerol ester(s) or a combination thereof;
preferably wherein said one or more glucose-ether(s) is methyl gluceth-20;
and/or wherein said one or more glycerol ester(s) is glyceryl oleate, glyceryl palmate, glyceryl cocoate, glyceryl undecylenate, or a combination of any of the foregoing.

12. The composition of any one of claims 9-11, wherein said one or more vegetable extract(s) is *Matricaria chamomilla, Aloe vera, Glycyrrhiza glabra L.* or a combination of any of the foregoing.

13. The composition of any one of claims 1 to 12, wherein said composition is a foaming composition and the composition further comprises a surfactant present in an amount of 1 to 5 % w/w of the composition;
preferably wherein said surfactant is an ethoxylated dimethicone having a comb structure or a terminal structure or a combination thereof;
more preferably wherein said surfactant is 3-(3- Hydroxypropyl)-heptamethyltrisiloxane; a polyether-modified polysiloxane; a polysiloxane betaine; an alkenyl ester or diester of sulfosuccinic acid in which the alkyl or alkenyl groups independently contain from six to eighteen carbon atoms; an alkali metal, triethanolamine, sodium, ammonium, calcium or magnesium C8 to C18 alkyl ether sulfate; a C8-C18 alkyl amine oxide; an alkylpolyglusoside; a water-soluble resin or a combination of any of the foregoing.

14. The composition of claim 13, wherein said water-soluble resin is a non- ionic polymer of ethylene oxide having a molecular weight from about 100,000 to about 4,000,000 Daltons;
preferably wherein said surfactant is Bis-PEG-12 dimethicone present in an amount of 1.00 % w/w of the composition.

15. The composition of any one of claims 1 to 14, wherein the composition is thickened by a rheology modifier present in amount of from 0.01 to 10 % w/w of the composition;
preferably wherein said rheology modifier is an acrylate/C 10-30 alkyl acrylate crosspolymer, carbomer, xanthan gum, guar gum, cellulose ether, quaternised guar gum, alginate, bentonite or a combination of any of the foregoing

## Patentansprüche

1. Hautdesinfektionsmittel-Zusammensetzung, umfassend:
(a) eine Menge von 60 bis 70 Gew.-% eines aliphatischen C2 bis C5-Alkohols, wobei der Alkohol Ethanol ist;
(b) einen oder mehrere Chelatbildner, der bzw. die in einer Menge von 0,1 bis 5 Gew.-% der Zusammensetzung vorhanden ist bzw. sind;
(c) ein oder mehrere Hydrotrop(e), das bzw. die in einer Menge von 0,1 bis 5,0 % Gew.-% der Zusammensetzung vorhanden sind;
(d) eine oder mehrere anorganische Säure(n) und/oder eine oder mehrere organische Säure(n), die in einer Menge von 0,01 bis 5 % Gew.-% der Zusammensetzung vorhanden ist bzw. sind und zur Einstellung des pH-Werts der Zusammensetzung in einem Bereich von ca. pH 2 bis ca. pH 6 ausgewählt ist bzw. sind;
(e) ein oder mehrere C10- bis C16-Alkylbenzolsulfonat(e), das bzw. die in einer Menge von 0,1 bis 1,0 Gew.-% der Zusammensetzung vorhanden ist bzw. sind;
(f) Harnstoff, Ammoniumsulfat oder eine Kombination davon, die in einer Menge von 0,01 bis 10 Gew.-% der Zusammensetzung vorhanden sind;
(g) ein oder mehrere Sesquiterpenoid(e), das bzw. die in einer Menge von 0,01 bis 1 Gew.-% der Zusammensetzung vorhanden ist bzw. sind;
(h) ein oder mehrere Hautfeuchthalte-, Hautkonditionier- und Hautpflegemittel, das bzw. die in einer Menge von 0,01 bis 10 % Gew.-% der Zusammensetzung vorhanden ist bzw. sind; und
(i) Wasser, das in ausreichender Menge vorhanden ist, um die Gesamtzusammensetzung auf 100 Gew.-% zu bringen.

2. Zusammensetzung nach Anspruch 1, bei der der eine bzw. die mehreren Chelatbildner Glutaminsäure, eine oder mehrere Phosphonsäure(n) mit 1 bis 5 Phosphonsäuregruppen oder ein Gemisch aus beliebigen dieser ist bzw. sind; bei der vorzugsweise der eine bzw. die mehreren Chelatbildner 1-Hydroxyethyliden-1,1-diphosphonsäure, Amino-tri(methylenphosphonsäure), Diethylentriaminpenta(methylenphosphonsäure), 2-Hydroxyethyl-iminobis(methylenphosphonsäure), Ethylendiamintetra(methylenphosphonsäure), Natriumtripolyphosphat, Ethylendiamintetraessigsäure (EDTA), Diethylentriaminpentaessigsäure (DTPA), N-(Hydroxyethyl)-ethylen-diamintriessigsäure (HEDTA), 2-Hydroxyethyliminodiessigsäure (HEIDA), Iminodibernsteinsäure, Polyasparaginsäure, Tetranatriumglutamatdiacetat oder ein Gemisch aus beliebigen der vorgenannten Substanzen ist.

3. Zusammensetzung nach Anspruch 2, bei der der eine bzw. die mehreren Chelatbildner Tetranatriumglutamatdiacetat ist.

4. Zusammensetzung nach einem der Ansprüche 1 bis 3, bei der das eine bzw. die mehreren Hydrotrop(e) Xylolsulfonsäure, Natriumxylolsulfonat, Ammoniumxylolsulfonat, Calciumxylolsulfonat, Kaliumxylolsulfonat, Toluolsulfonsäure, Natriumtoluolsulfonat, Ammoniumtoluolsulfonat, Cumolsulfonsäure, Natriumcumolsulfonat, Ammoniumcumolsulfonat, Phosphatpolyetherester, Alkyldiphenyloxiddisulfonat, ein Salz einer der vorstehenden Substanzen oder eine Gemisch einer der vorstehenden Substanzen ist; wobei vorzugsweise das eine bzw. die mehreren Hydrotrop(e) Cumolsulfonsäure oder deren Salz ist.

5. Zusammensetzung nach einem der Ansprüche 1 bis 4, bei der die eine bzw. die mehreren anorganische(n) Säure(n) und/oder die eine oder mehreren organische(n) Säure(n) eine oder mehrere alpha-Hydroxysäure, Maleinsäure, Propansäure, Butansäure, Pentansäure, Hexansäure, Heptansäure, eine Mono-Octansäure, eine Di-Octansäure, eine Tri-Octansäure, eine oder mehrere beta-Hydroxysäure(n), Phosphorsäure oder eine Kombination aus beliebigen dieser Substanzen ist;
wobei vorzugsweise die eine oder mehreren alpha-Hydroxysäure(n) Zitronensäure, Milchsäure, Weinsäure, Äpfelsäure, Glykolsäure oder eine Kombination aus beliebigen dieser Substanzen ist.

6. Zusammensetzung nach den Ansprüchen 1 bis 5, bei der das eine oder C10- bis C16-Alkylbenzolsulfonat Natriumdecylbenzolsulfonat, Natriumdodecylbenzolsulfonat, Natriumtridecylbenzolsulfonat oder eine Kombination aus beliebigen dieser Substanzen ist.

7. Zusammensetzung nach einem der Ansprüche 1 bis 6, bei der das eine bzw. die mehreren Sesquiterpenoid(e) Nerolidol, Farnesol, Bisabolol, Apriton oder eine Kombination aus beliebigen dieser Substanzen ist.

8. Zusammensetzung nach Anspruch 7, bei der das eine bzw. die mehreren Sesquiterpenoid(e) Nerolidol ist.

9. Zusammensetzung nach einem der Ansprüche 1 bis 8, bei der das eine bzw. die mehreren Hautfeuchthalte- und/oder Hautkonditionier- und/oder Hautpflegemittel ein oder mehrere Polyol(e) und Glykol(e), ein oder mehrere Phospholipid(e), eine oder mehrere essentielle Fettsäure(n), ein oder mehrere Vitamin(e), eine oder mehrere kationische(n) Verbindung(en), ein oder mehrere Zuckerderivat(e), ein oder mehrere Pflanzenextrakt(e) oder eine Kombination aus beliebigen dieser Substanzen ist.

10. Zusammensetzung nach Anspruch 9, bei der das eine bzw. die mehreren Phospholipid(e) Sojalecithin, ein Ceramid oder eine Kombination davon ist;
und/oder wobei die eine bzw. die mehreren essentielle(n) Fettsäure(n) Linolensäure, Gamma-Linolensäure, Linolsäure, Gamma-Linolsäure oder eine Kombination aus beliebigen dieser Substanzen ist;
und/oder wobei das eine bzw. die mehreren Vitamin(e) D-Panthenol, ein Tocopherol oder eine Kombination davon ist;
und/oder wobei die eine oder mehrere kationische(n) Verbindung(en) ein quaternisiertes Gummi, ein quaternisiertes Guargummi, Dihydroxypropyl-PEG-5-Linoleammoniumchlorid, Behentrimoniumchlorid, ein Polyquaternium oder eine Kombination aus beliebigen dieser Substanzen ist.

11. Zusammensetzung nach einem der Ansprüche 9 bis 10, bei der das eine bzw. die mehreren Zuckerderivat(e) ein oder mehrere Glucose-Ether, ein oder mehrere Glycerinester oder eine Kombination davon ist;
wobei vorzugsweise der eine bzw. die mehreren Glucose-Ether Methylgluceth-20 ist; und/oder wobei der eine bzw. die mehreren Glycerinester Glyceryloleat, Glycerylpalmat, Glycerylcocoat, Glyceryl Undecylenat oder eine Kombination aus beliebigen dieser Substanzen ist.

12. Zusammensetzung nach einem der Ansprüche 9 bis 11, bei der der eine bzw. die mehreren Pflanzenextrakt(e) Matricaria chamomilla, Aloe Vera, Glycyrrhiza glabra L. oder eine Kombination aus beliebigen dieser Substanzen ist.

13. Zusammensetzung nach einem der Ansprüche 1 bis 12, bei der die Zusammensetzung eine schäumende Zusammensetzung ist und die Zusammensetzung weiterhin ein Tensid umfasst, das in einer Menge von 1 bis 5 Gew.-% der Zusammensetzung vorhanden ist;
wobei vorzugsweise das Tensid ein ethoxyliertes Dimethicon mit kammartiger Struktur oder terminaler Struktur oder einer Kombination davon ist;
wobei das Tensid 3-(3-Hydroxypropyl)-heptamethyltrisiloxan; ein Polyether-modifiziertes Polysiloxan; ein Polysiloxanbetain; ein Alkenylester oder -diester von Sulfobernsteinsäure ist, bei dem die Alkyl- oder Alkenylgruppen unabhängig voneinander sechs bis achtzehn Kohlenstoffatome enthalten; ein Alkalimetall-, Triethanolamin-, Natrium-, Ammonium-, Calcium- oder Magnesium-C8- bis C18-Alkylethersulfat; ein C8 bis C18-Alkylaminoxid; ein Alkylpolyglucosid; ein wasserlösliches Harz oder eine Kombination aus beliebigen dieser Substanzen ist.

14. Zusammensetzung nach Anspruch 13, bei der das wasserlösliche Harz ein nicht-ionisches Polymer von Ethylenoxid mit einem Molekulargewicht von etwa 100.000 bis etwa 4.000.000 Dalton ist;
wobei vorzugsweise das Tensid Bis-PEG-12-Dimethicon ist, das in einer Menge von 1,00 Gew.-% der Zusammensetzung vorliegt.

15. Zusammensetzung nach einem der Ansprüche 1 bis 14, bei der die Zusammensetzung durch einen Rheologiemodifikator verdickt ist, der in einer Menge von 0,01 bis 10 Gew.-% der Zusammensetzung vorhanden ist;
wobei vorzugsweise der Rheologiemodifikator ein Acrylat/C10-30-Alkylacrylat-Kreuzpolymer, Carbomer, Xanthangummi, Guargummi, Celluloseether, quaternisiertes Guargummi, Alginat, Bentonit oder eine Kombination aus beliebigen dieser Substanzen ist.

## Revendications

1. Composition désinfectante pour la peau, comprenant :
(a) une quantité de 60 à 70 % p/p d'un alcool aliphatique en C2 à C5, dans laquelle l'alcool est l'éthanol
(b) un ou plusieurs agents chélateurs présents en une quantité de 0,1 à 5 % p/p de la composition ;
(c) un ou plusieurs hydrotropes, présents en une quantité de 0,1 à 5,0% p/p de la composition ;
(d) un ou plusieurs acides inorganiques et/ou un ou plusieurs acides organiques présents en une quantité de 0,01 à 5 % p/p de la composition, sélectionnés pour ajuster le pH de la composition dans une plage d'environ un pH 2 à environ à un pH 6 ;
(e) un ou plusieurs sulfonates d'alkylbenzène en C10 à C16 présents en une quantité de 0,1 à 1,0 % p/p de la composition ;
(f) de l'urée, du sulfate d'ammonium ou une combinaison de ceux-ci présents en une quantité de 0,01 à 10 % p/p de la composition ;
(g) un ou plusieurs sesquiterpénoïdes présents en une quantité de 0,01 à 1 % p/p de la composition ;
(h) un ou plusieurs agents d'hydratation, de conditionnement et agents émollients de la peau présents en une quantité de 0,01 à 10 % p/p de la composition ; et
(i) de l'eau est présente en une quantité pour équilibrer la composition totale à 100 % p/p.

2. Composition selon la revendication 1, dans laquelle lesdits un ou plusieurs agents chélateurs sont l'acide glutamique, un ou plusieurs acides phosphoniques ayant 1 à 5 groupes d'acide phosphonique, ou un mélange de l'un quelconque de ce qui précède ; de préférence dans laquelle lesdits un ou plusieurs agents chélateurs sont l'acide 1-hydroxyéthylidène-1,1,-diphosphonique, l'amino tri(acide méthylène phosphonique), le diéthylènetriaminepenta(acide méthylène phosphonique), le 2-hydroxy éthylimino bis(acide méthylène phosphonique), l'éthylène diamine tétra(acide méthylène phosphonique), le tripolyphosphate de sodium, l'acide éthylènediaminetétraacétique (EDTA), l'acide diéthylènetriaminepentaacétique (DTPA), l'acide N-(hydroxyéthyl)-éthylènediaminetriacétique (HEDTA), l'acide 2-hydroxyéthyliminodiacétique (HEIDA), l'acide iminodisuccinique, l'acide polyaspartique, le diacétate de glutamate de tétrasodium, ou un mélange de l'un quelconque de ce qui précède.

3. Composition selon la revendication 2, dans laquelle lesdits un ou plusieurs agents chélateurs sont le diacétate de glutamate de tétrasodium.

4. Composition selon l'une quelconque des revendications 1 à 3, dans laquelle lesdits un ou plusieurs hydrotropes sont l'acide xylène sulfonique, le xylène sulfonate de sodium, le xylène sulfonate d'ammonium, le xylène sulfonate de calcium, le xylène sulfonate de potassium, l'acide toluène sulfonique, le toluène sulfonate de sodium, le toluène sulfonate d'ammonium, l'acide cumène sulfonique, le cumène sulfonate de sodium, le cumène sulfonate d'ammonium, le polyéther ester de phosphate, le disulfonate d'alkyldiphényloxyde, un sel de l'un quelconque de ce qui précède ou un mélange de l'un quelconque de ce qui précède ;
de préférence dans laquelle lesdits un ou plusieurs hydrotropes sont l'acide cumène sulfonique ou son sel.

5. Composition selon l'une quelconque des revendications 1 à 4, dans laquelle lesdits un ou plusieurs acides inorganiques et/ou lesdits un ou plusieurs acides organiques sont un ou plusieurs acides alpha-hydroxy, acides maléiques, acides propanoïques, acides butanoïques, acides pentanoïques, acides hexanoïques, acides heptanoïques, un acide mono-octanoïque, un acide di-octanoïque, un acide tri-octanoïque, un ou plusieurs acides bêta-hydroxy, acides phosphoriques ou une combinaison de l'un quelconque de ce qui précède ;
de préférence dans laquelle lesdits un ou plusieurs acides alpha-hydroxy sont l'acide citrique, l'acide lactique, l'acide tartrique, l'acide malique, l'acide glycolique ou une combinaison de l'un quelconque de ce qui précède.

6. Composition selon les revendications 1 à 5, dans laquelle lesdits un ou plusieurs sulfonates d'alkylbenzène en C10 à C16 sont le décylbenzène sulfonate de sodium, le dodécylbenzène sulfonate de sodium, le tridécylbenzène sulfonate de sodium ou une combinaison de l'un quelconque de ce qui précède.

7. Composition selon l'une quelconque des revendications 1 à 6, dans laquelle lesdits un ou plusieurs sesquiterpénoïdes sont le nérolidol, le farnésol, le bisabolol, l'apritone ou une combinaison de l'un quelconque de ce qui précède.

8. Composition selon la revendication 7, dans laquelle lesdits un ou plusieurs sesquiterpénoïdes sont le nérolidol.

9. Composition selon l'une quelconque des revendications 1 à 8, dans laquelle lesdits un ou plusieurs agents d'hydratation et/ou de conditionnement ou agents émollients de la peau sont un ou plusieurs polyols et glycols, un ou plusieurs phospholipides, un ou plusieurs acides gras essentiels, une ou plusieurs vitamines, un ou plusieurs composés cationiques, un ou plusieurs dérivés de sucre, un ou plusieurs extraits végétaux ou une combinaison de l'un quelconque de ce qui précède.

10. Composition selon la revendication 9, dans laquelle lesdits un ou plusieurs phospholipides sont la lécithine de soja, une céramide ou une combinaison de l'une quelconque de ce qui précède ;
et/ou dans laquelle lesdits un ou plusieurs acides gras essentiels sont l'acide linolénique, l'acide gamma-linolénique, l'acide linoléique, l'acide gamma-linoléique ou une combinaison de l'un quelconque de ce qui précède ;
et/ou dans laquelle lesdites une ou plusieurs vitamines sont le d-panthénol, un tocophérol ou une combinaison de ce qui précède ;
et/ou dans laquelle lesdits un ou plusieurs composés cationiques sont une gomme quaternisée, une gomme de guar quaternisée, le chlorure de dihydroxypropyl PEG-5 linoleammonium, le chlorure de béhentrimonium, un polyquaternium ou une combinaison de l'un quelconque de ce qui précède.

11. Composition selon l'une quelconque des revendications 9 ou 10, dans laquelle lesdits un ou plusieurs dérivés de sucre sont un ou plusieurs éthers du glucose, un ou plusieurs esters de glycérol ou une combinaison de ceux-ci ;
de préférence dans laquelle lesdits un ou plusieurs éthers du glucose sont le méthyl gluceth-20 ;
et/ou dans laquelle lesdits un ou plusieurs esters de glycérol sont l'oléate de glycéryle, le palmate de glycéryle, le cocoate de glycéryle, l'undécylénate de glycéryle, ou une combinaison de l'un quelconque de ce qui précède.

12. Composition selon l'une quelconque des revendications 9 à 11, dans laquelle lesdits un ou plusieurs extraits végétaux sont la *Matricaria chamomilla,* l*'Aloe vera,* la *Glycyrrhiza glabra L.* ou une combinaison de l'une quelconque de ce qui précède.

13. Composition selon l'une quelconque des revendications 1 à 12, dans laquelle ladite composition est une composition moussante et la composition comprend en outre un tensioactif présent en une quantité de 1 à 5 % p/p de la composition ;
de préférence dans laquelle ledit tensioactif est un diméthicone éthoxylé ayant une structure en peigne ou une structure terminale ou une combinaison de celles-ci ;
de préférence encore dans laquelle ledit tensioactif est le 3-(3-Hydroxypropyl)-heptaméthyltrisiloxane ; un polysiloxane modifié par un polyéther ; une polysiloxane bétaïne ; un alcényl ester ou diester d'acide sulfosuccinique dans lequel les groupes alkyle ou alcényle contiennent indépendamment six à dix-huit atomes de carbones ; un métal alcalin, une triéthanolamine, un sulfate d'alkyl éther de sodium, ammonium, calcium ou magnésium en C8 à C18 ; un oxyde d'alkylamine en C8 à C18 ; un alkylpolyglucoside ; une résine hydrosoluble ou une combinaison de l'un quelconque de ce qui précède.

14. Composition selon la revendication 13, dans laquelle ladite résine hydrosoluble est un polymère non-ionique d'oxyde d'éthylène ayant un poids moléculaire d'environ 100 000 à environ 4 000 000 Daltons ;
de préférence dans laquelle ledit tensioactif est le Bis-PEG-12 diméthicone présent en une quantité de 1,00 % p/p de la composition.

15. Composition selon l'une quelconque des revendications 1 à 14, dans laquelle la composition est épaissie par un modificateur de rhéologie présent en une quantité de 0,01 à 10 % p/p de la composition ;
de préférence dans laquelle ledit modificateur de rhéologie est un polymère réticulé d'acrylate/acrylate d'alkyle en C10 à C30, un carbomère, une gomme de xanthane, une gomme de guar, un éther de cellulose, une gomme de guar quaternisée, un alginate, une bentonite ou une combinaison de l'un quelconque de ce qui précède.
